# EUROPÄISCHE PATENTSCHRIFT

(11) **EP 0 799 813 B1**
(45) Veröffentlichungstag und Bekanntmachung des Hinweises auf die Patenterteilung: **16.02.2000**
(21) Anmeldenummer: 97104730.3
(22) Anmeldetag: 20.03.1997
(51) Int. Cl.: C07C 15/28, C07D 209/84, C07C 15/30, C07C 13/567, C07C 50/18, C07D 209/86

(54) **Destillationsverfahren zur Verbesserung der Reinproduktengewinnung aus Rohanthracen**
Distillation process for the recovery of pure products from crude anthracene
Procédé de distillation pour la récupération de produits purs à partir d'anthracène brut

(30) Priorität: 04.04.1996 DE 19613497
(43) Veröffentlichungstag der Anmeldung: 08.10.1997
(73) Patentinhaber: VFT AG, 47138 Duisburg (DE)
(72) Erfinder: Fuhrmann, Edgar, Dr., 44579 Castrop-Rauxel (DE); Talbiersky, Jörg, Dr., 46282 Dorsten (DE); Erdmann, Wulf, 44579 Castrop-Rauxel (DE); Alsmeier, Freidhelm, 45133 Essen (DE)
(74) Vertreter: Cohausz & Florack

(56) Entgegenhaltungen:
- DE-C- 369 366
- DE-C- 550 310

## Beschreibung

Die Erfindung betrifft ein kontinuierliches Destillationsverfahren zur Verbesserung der Reinproduktengewinnung aus Rohanthracen.

Rohanthracen ist eine Kristallfraktion, die durch Suspensionskristallisation von Anthracenöl, einer Siedefraktion von etwa 300 bis 400°C der Rohteerraffination, gewonnen wird. Rohanthracen ist ein Gemisch verschiedener mehrkerniger Aromaten mit Phenanthren, Anthracen und Carbazol als Hauptbestandteilen neben vielen anderen Aromaten. Aufgrund dieser komplexen Zusammensetzung wird dieser Rohstoff nicht direkt zur Reinproduktenerzeugung durch Kristallisation eingesetzt, wenn mehrere Inhaltsstoffe gewonnen werden sollen.

Aus H.-G. Frank, J.W. Stadelhofer, Industrielle Aromatenchemie, Springer-Verlag (1987), S. 355/356, ist bekannt, Rohanthracen einer Destillation zu unterziehen und dabei eine Anreicherung des Anthracens von etwa 50% zu erreichen. Anschließend erfolgt die Aufarbeitung zu Reinanthracen mit einem Gehalt von über 95% durch Rekristallisation in polaren Lösungsmitteln.

Nachteilig an diesem Verfahren ist, daß das gewonnene Anthracen einen nennenswerten Gehalt an leichter siedenden Komponenten, insbesondere von Diphenylenoxid, aufweist, was in gewissen Anwendungsfällen störend wirkt.

Der zur destillativen Gewinnung der Anthracen- und Carbazolfraktion eingesetzte Rohstoff Rohanthracen enthält aufgrund seiner Herkunft hauptsächlich Carbazol als schwer siedende Komponente. Aus Gründen der Betriebssicherheit kann der bisherige Destillationssumpf nicht carbazolfrei gefahren werden, da die Kolonne mit einer Mindestsumpfabnahme gefahren werden muß, um Wärmeverluste in den Sumpfleitungen auszugleichen und um zu lange Verweilzeiten im Sumpf, die zu Verkokungen führen, zu unterdrücken.

Der Erfindung liegt somit die Aufgabe zugrunde, die obigen Nachteile zu vermeiden.

Gelöst wird diese Aufgabe durch ein Verfahren zur Verbesserung der Reinproduktengewinnung aus Rohanthracen, in dem man die Destillation zweistufig durchführt und in der ersten Stufe eine Fraktion eines Siedebereichs von 280 bis 320°C bei Normaldruck als Kopfprodukt abtrennt, dem Sumpf aus dieser Kolonne Carbazolrückstände zuführt und dieses Gemisch in einer zweiten Kolonne in Anthracen- und Carbazolfraktion auftrennt.

Das erfindungsgemäße Verfahren ermöglicht die Gewinnung einer anthracenfreien Diphenylenoxid/Fluoren-Fraktion als Kopfprodukt in der ersten Destillationsstufe. Diese Fraktion kann direkt in die Kristallisation zur Gewinnung von Fluoren eingesetzt werden. Weitere Vorteile bestehen darin, daß die Rückführung von Carbazolanteilen der Kristallisation zurück in die Destillation die Ausbeute an Reincarbazol, bezogen auf den Einsatz an Rohanthracen, ganz beträchtlich steigert, weil die Verluste der Kristallisation zu einem großen Teil wieder durch die Destillation aufgefangen werden. Ferner führt die Rückführung von Kristallisationsrückständen mit hohen Anteilen an Carbazol und Sumpfkomponenten zu einer Anteilen an Carbazol und Sumpfkomponenten zu einer Erhöhung der Carbazolkonzentration im Feed der Destillationskolonne und zu einer erhöhten Belastung des kritischen Abtriebteils der Kolonne. Bei Einhaltung der minimalen Sumpfabnahmemenge kann der Destillationssumpf heißer und damit carbazolärmer gefahren werden. Dadurch werden die Carbazolverluste über die Sumpfspeisung minimiert und damit die Carbazolausbeute des Verfahrens zusätzlich verbessert.

Gemäß einer bevorzugten Ausführungsform der Erfindung führt man einen Teil des Sumpfes der zweiten Destillationskolonne ab und speist diesen wieder in diese Destillationskolonne ein.

Gemäß einer weiteren bevorzugten Ausführungsform nimmt man die Phenanthren enthaltende Kopffraktion ab. Nach einer anderen bevorzugten Ausführungsform des erfindungsgemäßen Verfahrens wird eine anthracenfreie Diphenylenoxid/Fluoren-Fraktion als Kopfprodukt der ersten Destillationsstufe gewonnen. Vorzugsweise führt man die Destillation in Bodenkolonnen durch.

Die erfindungsgemäß eingesetzten Carbazolrückstände entstammen der Carbazolkristallisation, der die in der zweiten Destillationskolonne erhaltene Carbazolfraktion unterzogen wird. Diese enthalten neben etwa 40 Gew.% Carbazol überwiegend schwersiedende und damit sumpfbildende Bestandteile.

Nachfolgend werden die Produktqualitäten des erfindungsgemäßen Verfahrens mit denjenigen nach dem Stand der Technik verglichen.

| | Erfindung | Stand der Technik |
|---|---|---|
| DPO-Gehalt im Anthracen 50% | < 10 ppm | etwa 900 ppm |
| DPO-Gehalt im Reinanthracen | etwa 1ppm | etwa 70 ppm |
| Carbazolausbeute in der Destillation | etwa 75% | etwa 70% |
| Carbazolausbeute in der Kristallisation | etwa 66% | etwa 66% |
| Reincarbazolausbeute | etwa 66% | etwa 46% |

Diese Ergebnisse zeigen deutlich die Überlegenheit des erfindungsgemäßen Verfahrens.

Im Verfahrensfließbild 1 ist das erfindungsgemäße Verfahren schematisch erläutert, das Verfahrensfließbild 2 bezieht sich auf den Stand der Technik.

Nachfolgend wird eine beispielhafte Ausführungsform des erfindungsgemäßen Verfahrens beschrieben.

Schmelzflüssiges Rohanthracen wird als Feed in die Kolonne eingespeist und am Kopf dieser Kolonne eine Fraktion des Siedebereichs von 280 bis 320°C (bei Normaldruck) abgenommen. Diese anthracenfreie Fraktion kann direkt in die Kristallisation zur Gewinnung von Fluoren eingesetzt werden. Das von den Leichtsiedern befreite Sumpfprodukt der Kolonne 1 wird zusammen mit Carbazolrückständen, einem Nebenprodukt der Carbazolgewinnung, als Feed in die nachgeschaltete Kolonne 2 eingespeist. Weiterhin wird ein Teil des Sumpfs der Kolonne 2 als Feed in die Kolonne 2 zurückgeführt.

Am Kopf der Kolonne 2 wird eine Fraktion des Siedebereichs 320 bis 335°C (bei Normaldruck) abgenommen, die als Hauptbestandteil Phenanthren enthält.

Als Seitenströme werden die Anthracenfraktion mit einem Siedebereich von 335 bis 345°C (bei Normaldruck) und die Carbazolfraktion mit einem Siedebereich von 345 bis 370°C (bei Normaldruck) abgenommen.

Über den Sumpf der Kolonne 2 werden die Schwersieder sowie ein Teil des eingesetzten Carbazols herausgefahren. Die Einspeisung der Carbazolrückstände in die Kolonne 2 hat den Zweck, die Sumpfbelastung der Kolonne 2 mit Schwersiedern jenseits des Carbazols zu erhöhen und die Carbazolanteile dieses Stoffstroms für die Gewinnung der Carbazolfraktion zu nutzen. Die partielle Rückführung des Sumpfs der Kolonne 2 zurück in den Feed der Kolonne dient ebenfalls der Erhöhung der Belastung des Abtriebteils der Kolonne, durch die eine entsprechend höhere Belastung des Auftriebteils ermöglicht wird. Dadurch kann das Rückflußverhältnis am Kopf der Kolonne 2 erhöht werden, wodurch eine Steigerung der Trennleistung der Kolonne im Auftriebsteil erzielt wird.

Zur Gewinnung von Reinprodukten aus den Fraktionen der destillativen Rohanthracenaufarbeitung werden die Fraktionen, insbesondere die Fluoren, Anthracen und Carbazol enthaltenden Fraktion in eine Lösemittelkristallisation, vorzugsweise unter Verwendung von polaren Lösemitteln, eingesetzt. Solche Lösemittel sind beispielsweise Dimethylformamid, N-Methylpyrrolidon, Acetophenon und Cyclohexanon. Die Kristallisation kann in Rührkühlern im Batchbetrieb erfolgen.

Der Lösemittelanteil und die Kühlendtemperaturen in der Kristallisation werden in Abhängigkeit von den Stoffeigenschaften der Fraktionen und den gewünschten Produktreinheiten eingestellt. Zur Trennung der Reinprodukte von der Mutterlauge kann vorteilhafterweise eine Zentrifuge eingesetzt werden. Gegebenenfalls kann das Schleudergut der Zentrifuge einer nachgeschalteten Trocknung zur Entfernung der Lösemittelanteile in den Reinprodukten unterzogen werden.

Erfindungsgemäße Reinproduktzusammensetzungen sind solche von Fluoren, Anthracen und Carbazol. Eine Fluorenfraktion war nach dem bekannten einstufigen Destillationverfahren nicht erhältlich. Durch Lösungsmittelkristallisation erhaltenes Fluoren war in erheblichem Maß mit Anthracen verunreinigt, da dieses durch Kristallisation kaum abzutrennen war. Die erfindungsgemäß gewonnene Fluoren-Reinproduktzusammensetzung ist anthracenarm und enthält höchstens etwa 5 Gew.%, vorzugsweise 3 Gew.% und in besonders bevorzugter Weise weniger als 2 Gew.% Anthracen.

Die Anthracen enthaltende Reinproduktzusammensetzung enthält weniger als etwa 60 ppm, vorzugsweise etwa 1 bis 40 ppm Diphenylenoxid.

Die Carbazol enthaltende Reinproduktzusammensetzung enthält mehr als 94 Gew.%, vorzugsweise mehr als 96 Gew.% Carbazol.

Typische Zusammensetzungen von Reinprodukten, die durch Lösemittelkristallisation von Fraktionen der erfindungsgemäßen destillativen Rohanthracenaufarbeitung gewonnen wurden, sind nachfolgend dargestellt.

| **Fluoren** | |
|---|---|
| **Hauptkomponenten** | **Anteil in Gew.%** |
| Acenaphten | 1,25 |
| Diphenylenoxid | 0,86 |
| Fluoren | 95,43 |
| Phenanthren | 0,12 |
| Anthracen | 0,57 |

| **Anthracen** | |
|---|---|
| **Hauptkomponenten** | **Anteil in Gew.%** |
| Diphenylenoxid | ca. 1 ppm |
| Fluoren | ca. 20 ppm |
| Phenanthren | 1,69 |
| Anthracen | 96,68 |
| Carbazol | 0,89 |
| hochsiedene Verbindungen | 0,08 |

| **Carbazol** | |
|---|---|
| **Hauptkomponenten** | **Anteil in Gew.%** |
| Phenanthren | 0,001 |
| Anthracen | 0,63 |
| Carbazol | 96,87 |
| hochsiedende Verbindungen | 1,73 |

Durch die erfindungsgemäße zweistufige destillative Aufarbeitung des Rohanthracens konnte die Qualität der Reinproduktzusammensetzung bezüglich des Gehalts an Leichtsiedern deutlich verbessert werden. Als Leitkomponente für diese Verbindungen ist Diphenylenoxid anzusehen. Es kann somit eine Anthracenqualität aus dem Rohstoff Rohteer erzeugt werden, die im Hinblick auf die Verunreinigung mit Leichtsiedern mit Material aus der Anthracensynthese konkurrieren kann. Dadurch wird der Anwendungsbereich dieses erfindungsgemäß erhaltenen Produkts entscheidend verbessert. Das gilt insbesondere für den Einsatz im Holzaufschluß zur Zellstoffgewinnung.

### Beispiel

In die Kolonne 1 eines Durchmessers von etwa 2 m mit mindestens 20 Böden werden 2370 l/h Rohanthracen, schmelzflüssig bei etwa 280°C, eingespeist. Bei einem Rückflußverhältnis R/D von 50/1 und einem Kopfdruck von etwa 120 mbar werden am Kopf der Kolonne 1 etwa 260 l/h Fluorenfraktion abgezogen.

Das praktisch diphenylenoxidfreie Sumpfprodukt wird mit etwa 2110 l/h zusammen mit etwa 390 l/h schmelzflüssigen Carbazolrückständen als Feed in die Kolonne 2 eines Durchmessers von 2 m mit mindestens 60 Böden eingespeist. Ferner werden etwa 1000 l/h Sumpf der Kolonne 2 als Feed in diese Kolonne zurückgeführt. Bei einem Rückflußverhältnis R/D von 20/1 und einem Kopfdruck von etwa 300 mbar werden am Kopf der Kolonne 2 etwa 320 l/h Phenanthrenfraktion abgezogen. An der oberen Seitenabnahme der Kolonne 2 werden etwa 1070 l/h Anthracenfraktion und an der unteren Seitenabnahme der Kolonne 2 etwa 730 l/h Carbazolfraktion abgezogen.

Die anthracenfreie Fluorenfraktion, die Phenanthrenfraktion, die leichtsiedefreie Anthracenfraktion und die Carbazolfraktion werden anschließend in bekannter Weise durch Umkristallisation aufgearbeitet, so daß im Falle von Anthracen ein Produkt mit einem Diphenylenoxidgehalt < 1 ppm erzielt wird. Durch Oxidation des Anthracens kann diphenylenoxidfreies Anthrachinon gewonnen werden.

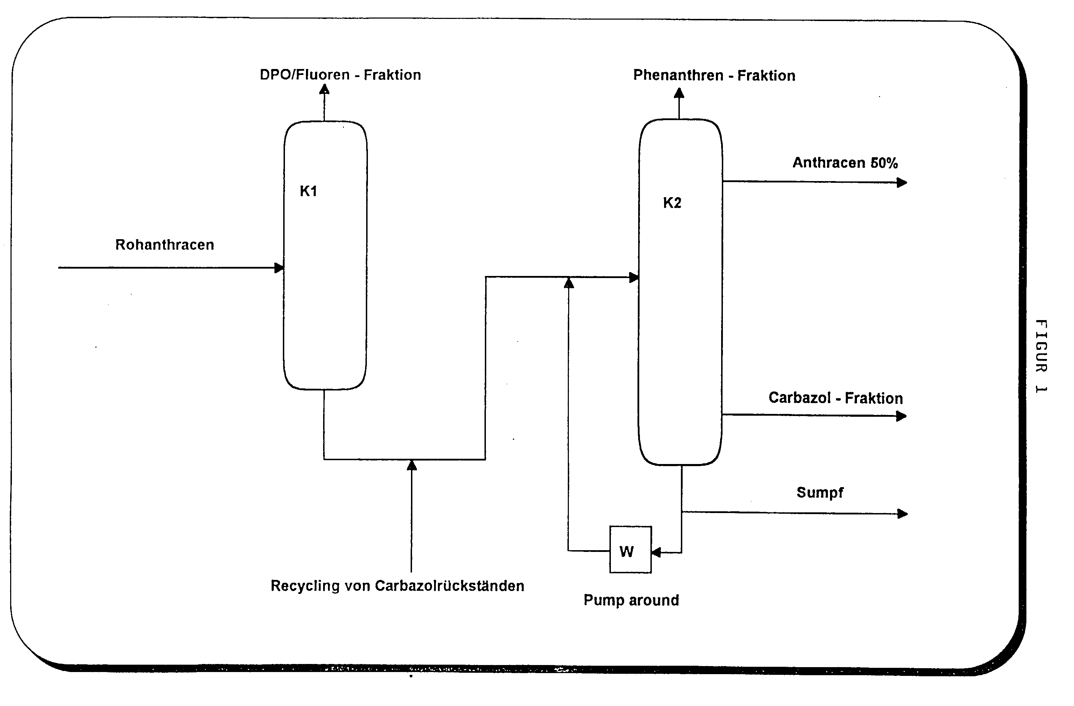

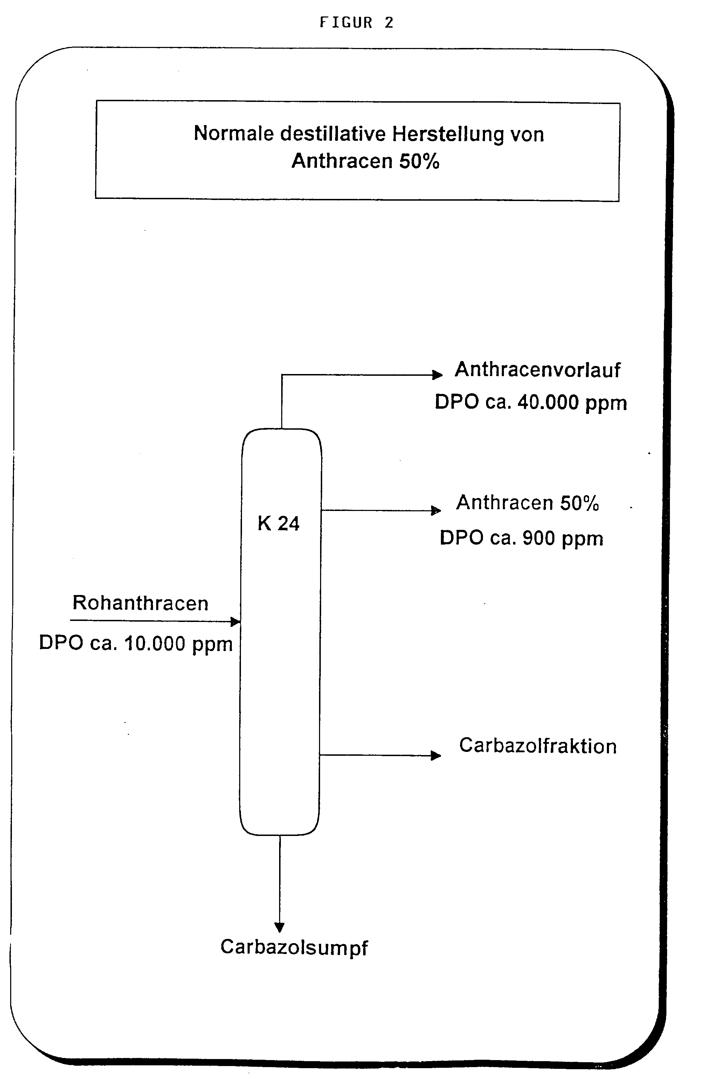

## Patentansprüche

1. Verfahren zur Verbesserung der Reinproduktengewinnung aus Rohanthracen, dadurch gekennzeichnet, daß man eine Destillation zweistufig durchführt und in der ersten Stufe eine Fraktion eines Siedebereichs von 280 bis 320°C bei Normaldruck als Kopfprodukt abtrennt, dem Sumpf dieser Kolonne Carbazolrückstände zuführt und dieses Gemisch in einer zweiten Kolonne in Anthracen- und Carbazolfraktion auftrennt.

2. Verfahren nach Anspruch 1, dadurch gekennzeichnet, daß man einen Teil des Sumpfes der zweiten Kolonne abführt und wieder in die Kolonne einspeist.

3. Verfahren nach einem der Ansprüche 1 oder 2, dadurch gekennzeichnet, daß man die Phenanthren enthaltende Kopffraktion abnimmt.

4. Verfahren nach einem der Ansprüche 1 bis 3, dadurch gekennzeichnet, daß man eine anthracenfreie Diphenylenoxid/Fluoren-Fraktion als Kopfprodukt der ersten Destillationskolonne abnimmt.

5. Verfahren nach einem der Ansprüche 1 bis 4, dadurch gekennzeichnet, daß man die Destillation in Bodenkolonnen durchführt.

6. Verfahren nach einem der Ansprüche 1 bis 5, dadurch gekennzeichnet, daß man die gewonnenen Anthracen, Carbazol, Phenanthren und Fluoren enthaltenden Fraktionen zum Erhalt von Anthracen, Carbazol, Phenanthren oder Fluoren aufarbeitet.

7. Verfahren nach Anspruch 6, dadurch gekennzeichnet, daß man die gewonnenen Anthracen, Carbazol, Phenanthren und Fluoren enthaltenden Fraktionen durch Lösungsmittelkristallisation aufarbeitet.

8. Verfahren nach Anspruch 1 bis 7, dadurch gekennzeichnet, daß man das so gewonnene Anthracen zu Anthrachinon oxidiert.

## Claims

1. A process for improving the yield of pure products from crude anthracene, characterized in that the distillation is carried out in two steps, and that in the first step a fraction from a boiling range of 280 to 320°C at normal pressure is separated as a top product; the bottom of this column is fed carbazole residues, and in a second column this mixture is separated into anthracene- and carbazole fractions.

2. A process according to claim 1, characterized in that part of the bottom of the second column is drained off and fed into the column again.

3. A process according to one of claims 1 or 2,
characterized in that the top fraction containing phenanthrene is removed.

4. A process according to one of claims 1 to 3,
characterized in that a diphenylene oxide/fluorene-fraction free of anthracene is removed as a top product of the first distillation column.

5. A process according to one of claims 1 to 4,
characterized in that the distillation is carried out in plate columns.

6. A process according to one of claims 1 to 5,
characterized in that the fractions obtained, which contain anthracene, carbazole, phenanthrene and fluorene, are processed to obtain anthracene, carbazole, phenanthrene or fluorene.

7. A process according to claim 6, characterized in that the fractions obtained, which contain anthracene, carbazole, phenanthrene and fluorene, are processed by solvent crystallisation.

8. A process according to claims 1 to 7, characterized in that the anthracene obtained in such a way is oxidised to anthraquinone.

## Revendications

1. Procédé d'amélioration de la récupération de produits purs à partir d'anthracène brut caractérisé en ce qu'une distillation est effectuée en deux étapes, en ce que dans la première étape une fraction du distillat entre 280 et 320 °C à pression normale est séparée comme produit de tête, en ce qu'on introduit un résidu de carbazole dans la pâte de cette colonne et en ce que le mélange est séparé dans une deuxième colonne en une fraction d'anthracène et de carbazole.

2. Procédé selon la revendication 1 caractérisé en ce qu'une partie de la pâte de la deuxième colonne est soustraite et à nouveau introduite dans la colonne.

3. Procédé selon une des revendications 1 ou 2, caractérisé en ce qu'on soustrait la fraction contenant le phénantrène.

4. Procédé selon une des revendications 1 à 3, caractérisé en ce qu'on soustrait une fraction de diphényloxyde-produits fluorés commme produit de tête de la première colonne de distillation.

5. Procédé selon une des revendications 1 à 4, caractérisé en ce qu'on effectue la distillation dans une colonne à plateaux.

6. Procédé selon une des revendications 1 à 5, caractérisé en ce que les fractions obtenues contenant l'anthracène, le carbazole, le phénantrène et les produits fluorés sont traités pour l'obtention d'anthracène, de carbazole, de phénantrène et de composés fluorés.

7. Procédé selon la revendication 6 caractérisé en ce que les fractions obtenues contenant l'anthracène, le carbazole, le phénantrène et les produits fluorés sont traités par cristallisation dans un solvant.

8. Procédé selon une des revendications 1 à 7, caractérisé en ce que l'anthracène obtenue est oxydée en anthracinone.
